# EUROPEAN PATENT APPLICATION

(11) **EP 3 545 762 A2**
(43) Date of publication of application: **02.10.2019**
(21) Application number: 19166341.8
(22) Date of filing: 29.03.2019
(51) Int. Cl.: A01N 25/04, A01N 37/46, A01N 37/44, A01N 47/44

(54) **FORMULATION AND COMPOSITION FOR PREVENTING AND/OR DISSOLVING BIOFILM ON THE SKIN OF A DOMESTIC ANIMAL**

(30) Priority: 30.03.2018 NL 2020701
(71) Applicant: Dechra Veterinary Products LLC, Overland Park, KS 66211 (US)
(72) Inventor: WIRTHERLE, Nicole Catherine, OVERLAND PARK, KS Kansas 66211 (US); ZOTTMAIER, Barbara, OVERLAND PARK, KS Kansas 66211 (US)
(74) Representative: Algemeen Octrooi- en Merkenbureau B.V.

(57) **Abstract**

The invention relates to a formulation for use in the prevention and/or dissolving of biofilm present on the skin of a domestic animal, said formulation comprising EDTA or a salt thereof and chlorhexidine or a derivative thereof, preferably said formulation also comprises N-acetylcysteine. The invention relates to a formulation for use in the prevention and/or dissolving of biofilm present on the skin of a domestic animal, said formulation comprising acetic acid and a second acid, being boric acid and/or lactic acid, preferably said formulation also comprises N-acetylcysteine.

## Description

### TECHNICAL FIELD

The present teaching relates to a formulation and a composition for preventing and/or dissolving biofilm on the skin of a domestic animal.

### BACKGROUND

The present teaching lies in the field of prevention, reduction and elimination of biofilms on animal skin. Especially, biofilms on the skin on the inside of an animal ear. Especially, on the skin of dogs, more preferably on the ear of a dog, e.g. for preventing or reducing otitis.

Most commercially available ear cleaners for dogs rely on softening and removing necrotic tissue and debris by using aqueous solutions of surfactants, optionally with preservatives and/or chelating agents. They are useful in cleaning the auditory canal of animals like dogs and cats from debris.

Biofilms are produces by many bacteria. A biofilm is a matrix of carbohydrates, proteins and polysaccharides which protects adherent bacteria on surfaces. Formation of biofilm is complex and essentially results in irreversible binding of a community of micro-organisms to a surface, such as the skin.

Biofilms allow bacteria to resists antibiotics and avoid the animals defence mechanism, providing a shelter for the bacteria. Biofilms have been noted in otitis. Otitis Externa is inflammation of the external ear canal distal to the tympanic membrane. Bacteria can survive within the protective constituents of the biofilm which also helps to prevent adequate penetration of topical and systemic treatment. Where biofilms are present and bacteria show a pattern of multiple resistance, treatment with conventional licensed drugs is often unsuccessful.

Chronic disease and long term use of antibiotics can lead to the development of multi-resistant bacterial infections with organisms such as *Pseudomonas aeruginosa.* Approximately 40% *of P. aeruginosa* isolates produce biofilms. In dogs, the most common skin and ear pathogens are *Staphylococcus pseudintermedius, Malassezia pachydermatis* and *Pseudomonas aeruginosa,* all of which are known to produce biofilms.

Research into biofilm formation, treatment and prevention in veterinary medicine is sparse despite an estimated prevalence in 65% of infections. *Pseudomonas* spp have been found to produce a mature biofilm within 10 hours.

There is a need for an improved formulation and composition that can remove biofilm from or reduce biofilm on skin of domestic animals.

### SUMMARY

It is an object of the present invention to provide an improved composition and/or formulation that can prevent and/or dissolve biofilm on skin of domestic animals.

It is a further object of the present invention to provide an improved composition and/or formulation that can prevent and/or dissolve biofilm on the ear canal of dogs.

The present teaching relates in a first aspect to a composition and/or formulation for use in the prevention and/or dissolving of biofilm present on the skin of a domestic animal, said composition and/or formulation comprising ethylene diamine tetra acetic acid (EDTA) or a salt thereof and chlorhexidine or a derivative thereof.

The present teaching relates in a second aspect to a composition and/or formulation for use in the prevention and/or dissolving of biofilm present on the skin of a domestic animal, said composition and/or formulation comprising acetic acid and a second acid, being boric acid and/or lactic acid.

A formulation according to the present teachings is a (preferably aqueous) dilution of the present composition. In other words, the composition may be used for preventing and/or dissolving of biofilm or a dilution thereof - the formulation - may be used for the prevention and/or dissolving of biofilm.
Several embodiments of the first and/or second aspects of the present teachings are shown below. Corresponding embodiments of the first aspect are also applicable for the second aspect and vice versa.

### LIST OF DEFINITIONS

The following definitions are used in the present description and claims to define the stated subject matter. Other terms not cited below are meant to have the generally accepted meaning in the field.
"bacteria" as used in the present description means: microscopic living organisms, usually one-celled. Bacteria can be pathogens, and as such cause infections. A common bacterium is *Pseudomonas aeruginosa.*
"biofilm" as used in the present description means: an aggregate of microorganisms, such as bacteria, in which cells that are frequently embedded within a self-produced matrix of extracellular polymeric substances adhere to each other and/or to a surface.
"skin" as used in the present description means: the soft outer tissue covering vertibrates. In mammals, the skin is an organ of the integumentary system made up of multiple layers of ectodermal tissue, and guards the underlying muscles, bones, ligaments and internal organs. The skin plays a key role in protecting the body against pathogens.
"domestic animal" as used in the present description means: an animal that has been tamed and kept by humans as a work animal, food source, or pet, especially a member of those species that have, through selective breeding, become notably different from their wild ancestors. Examples of domestic animals are dogs, cats and horses.
"ear canal" as used in the present description is also known as the external acoustic meatus, external auditory meatus or the (external) auditory canal, and means the tube running from the outer ear to the middle ear, distal to the tympanic membrane or ear drum.
"n-acetylcysteine" or "NAC" as used in the present description is the N-acetyl derivative of cysteine, and is also known by its IUPAC name (2R)-2-acetamido-3-sulfanylpropanoic acid.
"tris(hydroxymethyl)aminomethane" or "Tris" as used in the present description is also known during medical use as tromethamine or THAM, and is an organic compound with the formula (HOCH₂)₃CNH₂. It is also known by its preferred IUPAC name 2-amino-2-(hydroxymethyl)propane-1,3-diol.
"optical density" or "OD" as used in the present description is also known as absorbance, and expresses an estimation of the concentration of bacterial or other cells in a liquid. "OD₅₇₀" is the abbreviation indicating the absorbance, or optical density, of a sample measured at a wavelength of 570 nm.
"dilution" as used in the present description relates to a dilution factor, which describes the ratio of the volume of the liquid of interest to the final volume where a certain amount of solvent is added. For example, in a 1:4 dilution, with a 1:4 dilution factor, entails combining 1 unit volume of the solute (the material to be diluted) with (approximately) 3 units volumes of the solvent to give 4 units of total volume. This thus relates to a dilution *ratio* of 1:3.
"MIC" or "minimal inhibitory concentration" as used in the present description means: the lowest concentration of a chemical which prevents visible growth of a bacterium. This is in difference to the minimum bactericidal concentration (MBC) which is the concentration resulting in microbial death as defined by the inability to re-culture bacteria. An MIC depends on the microorganism, the chemical, and (in vivo only) the affected individual (animal).
"BPC" or "biofilm prevention concentration" is the minimal concentration of a chemical able to prevent the formation of biofilm.

### Embodiments of the first aspect

In an embodiment of said first aspect, said composition and/or formulation is for preventing of the formation of biofilm. In an embodiment of said first aspect, said composition and/or formulation is for dissolving biofilm.

In an embodiment of said first aspect, said composition and/or formulation comprises EDTA or a salt thereof and chlorhexidine or a derivative thereof and N-acetylcysteine. In an embodiment of said first aspect, the composition and/or formulation further comprising tris(hydroxymethyl)aminomethane (Tris). In an embodiment, the composition and/or formulation comprises a Tris-EDTA buffer solution. In an embodiment of said first aspect, EDTA is present in the form of the disodium salt of EDTA.

In an embodiment of said first aspect, the domestic animal is a dog. In an embodiment of said first aspect, the composition and/or formulation is for use in the ear canal.

In an embodiment of said first aspect, the composition and/or formulation is an liquid aqueous composition. In an embodiment of said first aspect, the composition and/or formulation is an ototopical solution. In an embodiment of said first aspect, the composition and/or formulation is an ear drop.

In an embodiment of the present invention, the formulations according to the inventions comprise a mixture of the composition and a solvent, preferably a broth solution or water. In other words, the formulations according to the teaching are dilutions of compositions. An example of a suitable dilution factor for the formulations are 1:2; viz. 1 part of the composition and 1 part of solvent (e.g. a broth solution or water) making 2 parts in total. An example of a suitable dilution factor for the formulations are 1:8; viz. 1 part of the composition and 7 part of solvent (e.g. broth or water) making 8 parts in total. Examples of dilutions factors for the formulation are: 1:x, wherein x is 2, 3, 4, 5, 6, 7, 8, 9, 10, and any number between 10 and 100. Specific examples are 1:2, 1:4, 1:8, 1:16, 1:32, 1:64, 1:128, and 1:256.

In an embodiment of said first aspect, the composition and/or formulation has a pH in the range of 6 to 9, preferably 6.0 to 9.0, for example between 7.0 and 8.5, such as between 7.8 to 8.2.

In an embodiment of said first aspect, one or more pH adjusting agents may be present in the composition and/or formulation, for example tris(hydroxymethyl)aminomethane hydrochloride (commercially known as Trizma® hydrochloride) in order to adjust the pH to the desired level.

In an embodiment of said first aspect, the composition and/or formulation comprises water, preferably at least 90 wt.% of water, more preferably at least 95 wt.% of water, such as at least 97 wt.% of water.

In an embodiment of said first aspect, the composition comprises tris(hydroxymethyl)aminomethane in an amount of between 0.002 and 5 wt. %, such as between 0.003 and 1 wt.%, preferably between 0.005 and 0.5 wt.%.

In an embodiment of said first aspect, the formulation comprises tris(hydroxymethyl)aminomethane in an amount of between 0.0001 wt.% and 2.5 wt.%, preferably between 0.0003 and 0.25 wt.%.

In an embodiment of said first aspect, the composition comprises EDTA (preferably as di-sodium) in an amount of between 0.002 and 0.5 wt. %, such as between 0.003 and 0.3 wt.%, preferably between 0.005 and 0.2 wt.%.

In an embodiment of said first aspect, the formulation comprises EDTA (preferably as di-sodium) in an amount of between 0.0001 and 0.25 wt. %, preferably between 0.0003 and 0.1 wt.%.

In an embodiment of said first aspect, the composition comprises chlorhexidine (preferably as digluconate) in an amount of between 0.01 and 5 wt. %, preferably between 0.05 and 1.0 wt.%, preferably between 0.1 and 0.2 wt.%.

In an embodiment of said first aspect, the formulation comprises chlorhexidine (preferably as digluconate) in an amount of between 0.0001 and 2.5 wt. %, preferably between 0.0005 and 0.5 wt.%, preferably between 0.01 and 0.1 wt.%.

### Embodiments of the second aspect

In an embodiment of the second aspect, the composition and/or formulation also comprises N-acetylcysteine.

In an embodiment of said second aspect, the composition and/or formulation is for preventing of the formation of biofilm. In an embodiment of said second aspect, said composition and/or formulation is for dissolving biofilm. In an embodiment of said second aspect, the domestic animal is a dog. In an embodiment of said second aspect, the composition and/or formulation is for use in the ear canal. In an embodiment of said second aspect, the composition and/or formulation is an liquid aqueous composition and/or formulation. In an embodiment of said second aspect, the composition and/or formulation is an ototopical solution. In an embodiment of said second aspect, the composition and/or formulation is an ear drop.

In an embodiment of said second aspect, the composition and/or formulation has a pH in the range of 2 to 5, more specifically in the range of 2.0 to 5.0, such as at least 2.5 or at least 3.0. In an embodiment, the composition and/or formulation has a pH in the range 4.4 to 4.8. In an embodiment of said second aspect, one or more pH adjusting agents may be present in the composition, for example selected from the group consisting of sodium hydroxide.

In an embodiment of said second aspect, the composition comprises water, preferably at least 75 wt.%, preferably at least 80 wt.% of water

In an embodiment of said second aspect, the composition comprises glycerine (also known as 1,2,3-propanetriol) in an amount of between 0.5 and 20 wt %, such as between 1 and 10 wt.% or between 2 and 8 wt.%.

In an embodiment of said second aspect, the formulation comprises glycerine (also known as 1,2,3-propanetriol) in an amount of between 0.005 and 10 wt. %, such as between 0.1 and 5 wt.% or between 0.1 and 2 wt.%.
In an embodiment of said second aspect, the composition comprises an emulsifier or solubilizing agent, such as polysorbates, which are derived from ethoxylated sorbitan (a derivative of sorbitol) esterified with fatty acids (for example the commercially known products Polysorbate® 20 or Tween® 20).

In an embodiment of said second aspect, the composition comprises a polysorbate in an amount of between 0.5 and 20 wt. %, such as between 1 and 10 wt.% or between 2 and 8 wt.%.

In an embodiment of said second aspect, the formulation comprises a polysorbate in an amount of between 0.005 and 10 wt. %, such as between 0.1 and 5 wt.% or between 0.1 and 2 wt.%.

In an embodiment of said second aspect, the composition comprises acetic acid in an amount of between 0.1 and 10 wt. %, such as between 0.5 and 5 wt.%, preferably between 1 and 3 wt.%.

In an embodiment of said second aspect, the formulation comprises acetic acid in an amount of between 0.001 or 0.005 and 5 wt. %, such as between 0.05 and 2.5 wt.% or between 0.05 and 2 wt.%.

In an embodiment of said second aspect, the composition comprises boric acid in an amount of between 0.0 and 10 wt. %, such as between 0.1 or 0.5 and 5 wt.%, preferably between 1 and 3 wt.%.

In an embodiment of said second aspect, the formulation comprises boric acid in an amount of between 0.0 or 0.005 and 5 wt. %, such as between 0.05 and 2.5 wt.%.

In an embodiment, the composition and/or formulation comprises a combination of acetic acid and boric acid.

In an embodiment of said second aspect, the composition comprises lactic acid in an amount of between 0.0 and 5 or between 0.0 and 2 wt. %, such as between 0.01 or 1 wt.%, for example between 0.05 and 0.5 wt.%.

In an embodiment of said second aspect, the formulation comprises lactic acid in an amount of between 0.0 and 2.5 wt. %, such as between 0.001 and 1 wt.%, or between 0.05 and 0.25 wt.%.

In an embodiment, the composition and/or formulation comprises a combination of acetic acid and lactic acid.

### DESCRIPTION OF EMBODIMENTS

The compositions according to the present teaching comprise one or more chelating agents, that is according to the first aspect ethylene diamine tetra acetic acid (EDTA) and according to the second aspect a combination of two acids, being acetic acid and either lactic acid or boric acid or both. A combination of one or more of these chelating agents may also be used for each aspect of the present teaching. The chemical structures of each of these chelating agents are shown below.

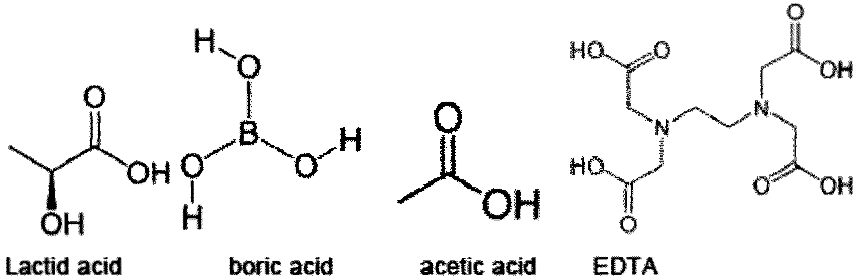

The composition according to the first aspect of the present teaching also comprises an antiseptic agent, chlorhexidine (1,1'-Hexamethylenebis[5-(4-chlorophenyl)biguanide]) (structure below) or a derivative thereof. Examples of derivatives are chlorhexidine gluconate, chlorhexidine digluconate (see structure below), chlorhexidine acetate, chlorhexidine diacetate, chlorhexidine hydrochloride, chlorhexidine dihydrochloride. Other derivates may also be used.

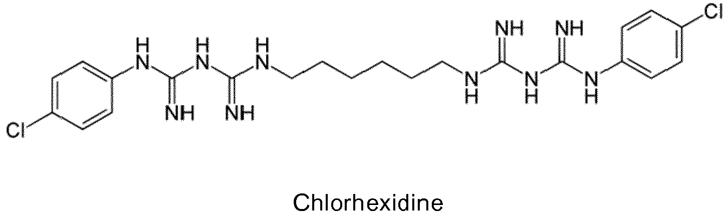

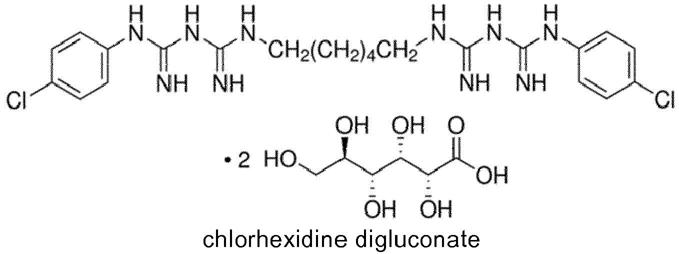

The composition according to the first or second aspect of the present teaching may further comprise N-acetylcysteine (NAC) (see structure below). NAC is normally used as a mycolytic agent.

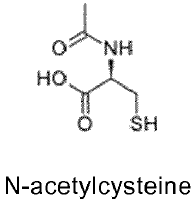

In a specific embodiment of the first aspect of the present invention, the composition comprises water (at least 95 wt.%), disodium EDTA, tris(hydroxymethyl) aminomethane and chlorhexidine digluconate.

In a specific embodiment of the first aspect of the present invention, the composition comprises water (at least 95 wt.%), disodium EDTA, tris(hydroxymethyl) aminomethane, chlorhexidine digluconate and N-acetylcysteine.

In a specific embodiment of the second aspect of the present invention, the composition comprises water (at least 80 wt.%), glycerine, polysorbate, acetic acid and boric acid.

In a specific embodiment of the second aspect of the present invention, the composition comprises water (at least 80 wt.%), glycerine, polysorbate, acetic acid, boric acid and N-acetylcysteine.

In a specific embodiment of the second aspect of the present invention, the composition comprises water (at least 80 wt.%), glycerin, polysorbate, acetic acid and lactic acid.

In a specific embodiment of the second aspect of the present invention, the composition comprises water (at least 80 wt.%), glycerin, polysorbate, acetic acid,lactic acid and N-acetylcysteine.

Other variations to the disclosed embodiments can be understood and effected by those skilled in the art in practicing the claimed teaching, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage.

The scope of the present teaching is defined by the appended claims. One or more of the objects of the teaching are achieved by the appended claims.

### EXAMPLES

The present teaching is further elucidated based on the Examples below which are illustrative only.

### Bacterial Isolates

One hundred *Pseudomonas* spp isolates from clinical canine otitis cases from the UK (96), Dubai (3) and Malta (1) were submitted to NationWide Laboratories Knutton (CAPL) for routine culture and susceptibility testing over a 12 month period. Four isolates were from two dogs with bilateral otitis. All isolates were susceptibility tested using a Microtitre plate MIC methodology (Sensititre®, ThermoFisher™, Basingstoke, UK) and applying CLSI criteria for interpretation (M100, performance standards for antimicrobial susceptibility testing, 28^{th} edition). Isolates were then stored frozen of beads (ProLab UK) at -20°C.

### Speciation

Isolates of *Pseudomonas* spp were speciated using the API® 20 NE system (Biomerieux, Basingstoke UK), in accordance with the manufacturer's instructions.

### MIC Determination

MIC was determined using a commercially available microbroth dilution system, Sensititre® (ThermoFisher™), according to the manufacturers guidelines and applying CLSI criteria for interpretation (M100, performance standards for antimicrobial susceptibility testing, 28^{th} edition). Briefly, individual isolates were added at a standardised inoculum density to wells of 96-well Microtitre plates that had had been pre-coated with the antimicrobial agents being tested at various concentrations around their break point. Due to innate resistance of *Pseudomonas,* enrofloxacin, gentamicin marbofloxacin, neomycin and pradofloxacin were considered relevant antimicrobials.

### Biofilm Assay

Isolates that had been stored on ProLab® beads were removed from - 20°C storage and sub-cultured onto blood agar plates for 24 hours. Biofilm assay was performed using microtitre plate assay (Stepanovic et al., Apmis 2007, 115(8), 891-899). Pure growth was inoculated in distilled, sterile water to 0.5McFarland (Sensititre® Nephelometer V3011) equating to 1.5x10⁸ colony forming units/ml (CFU). Aliquots of 20µL of each of 100 isolates (or a part of these 100 isolates) were added in triplicate to wells of a 96 well flat-bottomed microtitre plate (Thermo-Fisher™) containing 180µL of either Luria-Bertani enrichment broth (LBB), Mueller Hinton Broth (MHB) or Trypsin Soya Broth (TSB). Each plate contained only one broth. Negative control wells, in triplicate, contained 180µL of selected broth plus 20µL of distilled, sterile water.

After either 18 or 24 hours of incubation at 37°C, crystal violet assay was performed (Stepanovic et al, 2007). Contents of the wells were discarded prior to washing three times in phosphate buffered saline (PBS)at pH 7.2. Plates were inverted and heat fixed at 60°C for 60mins. 150µL of 0.1% crystal violet was then added to each well for 15mins. The contents were removed by micropipetting before plates rinsed thoroughly under running water. Plates were inverted and dried at 35°C for 15-30mins. 150µL of 95% ethanol was added to each well to elute the crystal violet staining. Plates were covered to prevent evaporation. After 30 mins at room temperature optical density (OD) of each well was read at 570nm (Thermo-Fisher™ Multiskan™ FC357) using Skanlt™ software. The triplicate mean OD₅₇₀ of the negative control was calculated for each plate and subtracted from the triplicate mean of each isolate in each separate broth to give a corrected OD₅₇₀. Biofilm production was classified as: no biofilm production if OD₅₇₀ was <0.135; low if 0.135-0.27; moderate if 0.27-0.54 and high if above 0.54 in agreement with previous studies (Pye et al. Veterinary Dermatology, 2013; 24: 446-449).

### Compositions used

An acetic acid/lactic acid composition (second aspect of invention) was used that is a clear, colourless liquid having a characteristic acetic/lemongrass-lavender odour. It has a pH of between 4.40 and 4.90 and consists of: 5.00 wt.% of glycerine as humectant, 5.00 wt.% of polysorbate® 20 as a polysorbate solubilizing agent, 2.00 wt.% of acetic acid and 0.10 wt.% of L-lactic acid as keratolytic agents and antiseptic agents, 0.08 wt.% of lemongrass oil and 0.05 wt.% of lavender oil as fragrances and 0.08 wt.% of sodium hydroxide as pH adjustment agent. The remaining being water.

An acetic acid/boric acid composition (second aspect of invention) was used that is a clear, colourless liquid having a characteristic acetic/apple door. It has a pH of between 4.4 and 4.8 and consists of: 5.00 wt.% of glycerine, 5.00 wt.% of polysorbate® 20, 2.00 wt.% of acetic acid and 2.00 wt.% of boric acid, 0.7 wt.% of an apple fragrance and the required amount of sodium hydroxide as pH adjustment agent. The remaining being water. This composition is commercially available as Malacetic Aural Apple®.

A Tris/EDTA/chlorhexidine composition (first aspect of invention) was used that is a clear, colourless liquid having no specific odour. The active ingredient is disodium EDTA and chlorhexidine digluconate. It has a pH of between 7.80 and 8.20 and consists of: 0.150 wt.% of chlorhexidine digluconate; 0.006 wt.% of tris(hydroxymethyl)aminomethane (also known as Tris or tromethamanine) and 0.005 wt.% of disodium EDTA and the required amount of tris(hydroxymethyl)aminomethane hydrochloride (commercially known as Trizma® hydrochloride) as pH adjustment agent. The remaining (approx. 99 wt.%) being water. This composition is commercially available as Trizchlor®.

A Tris/EDTA composition (not according to invention) was used that is a clear, colourless liquid having no specific odour. The active ingredient is disodium EDTA. It has a pH of between 7.80 and 8.20 and consists of: 0.45 wt.% of tris(hydroxymethyl)aminomethane and 0.119 wt.% of disodium EDTA and the required amount of tris(hydroxymethyl)aminomethane hydrochloride as pH adjustment agent. The remaining (approx. 99 wt.%) being water. This composition is commercially available as TrizAural®.

Since such a large portion of the compositions and formulations used are made up of water, wt.% and vol. % may be used interchangeably in the description.

### Biofilm prevention assay

Pure growth was inoculated in distilled, sterile water to 0.5 McFarland (Sensititre® Nephelometer V3011) equating to 1.5x108 colony forming units/ml (CFU). Aliquots of 20µL of each of the 28 isolates, selected using a random number generator, were added in triplicate to wells of a 96 well flat-bottomed microtitre plate (Thermo-Fisher™) containing 80µL of Luria-Bertani enrichment broth (LBB) and 100µL of compound (ear cleaner or n-acetyl cysteine). Negative control wells, in triplicate, contained 80µL of LBB, 100µL of compound and 20µL of distilled, sterile water. This (20µL isolate, 80µL broth and 100µL compound) is considered to be the dilution with dilution factor 1:2 (see the next section).

### Compositions and strengths tested:

For the biofilm prevention assay the following dilutions where tested. The dilutions were made using a Broth (LBB). Dilutions can give a more accurate indication of the *in vivo* scenario, e.g. application in a dog's ear, since there might be already moisture present that dilutes the application composition/formulation.
- Acetic acid/lactic acid composition: dilution factors 1:2, 1:4 and 1:8 (with and without varying amounts of NAC).
- Acetic acid/boric acid composition: dilution factors 1:2, 1:4, 1:8, 1:16 and 1:32 (with and without varying amounts of NAC).
- Tris/EDTA/chlorhexidine composition: dilution factors 1:2, 1:4, 1:8, 1:16, 1:32, 1:64 and 1:128 (with and without varying amounts of NAC).
- NAC (all mg/ml): 200, 100, 50, 20, 10, 5 and 2.5.

After 24 hours of incubation at 37°C, plates were assessed visually for MIC of each of the compounds tested and at various strengths before crystal violet assay was performed (Stepanovic et al, 2007). Contents of the wells were discarded prior to washing three times in phosphate buffered saline (PBS)at pH 7.2. Plates were inverted and heat fixed at 60oC for 60mins. 150µL of 0.1% crystal violet was then added to each well for 15mins. The contents were removed by micropipetting before plates rinsed thoroughly under running water. Plates were inverted and dried at 35°C for 15-30mins. 150µL of 95% ethanol was added to each well to elute the crystal violet staining. Plates were covered to prevent evaporation. After 30mins at room temperature optical density (OD) of each well was read at 570nm (Thermo-Fisher™ Multiskan™ FC357) using Skanlt™ software. The triplicate mean OD₅₇₀ of the negative control was calculated for each plate and subtracted from the triplicate mean of each isolate in each separate broth to give a corrected OD₅₇₀. Biofilm production was classified as: no biofilm production if OD₅₇₀ was <0.135; low if 0.135-0.27; moderate if 0.27-0.54 and high if above 0.54 in agreement with previous studies (Pye et al, 2013).

### Biofilm breaking assay

Pure growth was inoculated in distilled, sterile water to 0.5McFarland (Sensititre® NephelometerV3011) equating to 1.5x10⁸ colony forming units/ml (CFU). Aliquots of 20µL of each of the 14 isolates, selected using a random number generator, were added in triplicate to wells of a 96 well flat-bottomed microtitre plate (Thermo-Fisher™) containing 180µL of Luria-Bertani enrichment broth (LBB). Negative control wells, in triplicate, contained 180µL of LBB and 20µL of distilled, sterile water. Plates were then incubated for 24 hours at 37°C.
Contents of the wells were discarded prior to washing three times in phosphate buffered saline (PBS) at pH 7.2. To each well the following was added:
1. 100µL of Luria-Bertani enrichment broth (LBB) and 50µL compound (ear cleaner) and 50µL of distilled sterile water for ear cleaners only.
2. 100µL of Luria-Bertani enrichment broth (LBB) and 50µL of NAC with 50µL sterile distilled water for NAC only assessment.
3. 100µL of Luria-Bertani enrichment broth (LBB) and 50µL compound (ear cleaner) and 50µL of NAC for combination assessment.
4. 100µL of Luria-Bertani enrichment broth (LBB) and 100µL of sterile, distilled water for positive control was placed into wells that had previously contained isolate. The aim of this was to create a 48hour biofilm.
5. 100µL of Luria-Bertani enrichment broth (LBB) and 100µL of sterile, distilled water for negative control.

This (100µL broth and 50 µL compound and 50 µL water or NAC) is considered to be the dilution with dilution factor 1:4 (see the next section).

### Compositions and strengths tested:

For the biofilm breaking assay the following dilutions where tested. The dilutions were made using a Broth (LBB). Dilutions can give a more accurate indication of the *in vivo* scenario, e.g. application in a dog's ear, since there might be already moisture present that dilutes the application composition/formulation.
- Acetic acid/lactic acid: 1:4, 1:8, 1:16, 1:32 and 1:64 (with and without varying amounts of NAC)
- Acetic acid/boric acid: 1:4, 1:8, 1:16, 1:32 and 1:64 (with and without varying amounts of NAC)
- Tris-EDTA-chlorhexidine: 1:4, 1:8, 1:16, 1:32, 1:64, 1:128 and 1:256 (with and without varying amounts of NAC)
- NAC (all mg/ml): 20, 10, 5 and 2.5.

Plates were then incubated for another 24 hours period. Contents of the wells were discarded prior to washing three times in phosphate buffered saline (PBS) at pH 7.2. Plates were inverted and heat fixed at 60°C for 60mins. 150µL of 0.1% crystal violet was then added to each well for 15mins. The contents were removed by micropipetting before plates rinsed thoroughly under running water. Plates were inverted and dried at 35°C for 15-30mins. 150µL of 95% ethanol was added to each well to elute the crystal violet staining. Plates were covered to prevent evaporation. After 30mins at room temperature optical density (OD) of each well was read at 570nm (Thermo-Fisher™ Multiskan™ FC357) using Skanlt™ software. The triplicate mean OD₅₇₀ of the negative control was calculated for each plate and subtracted from the triplicate mean of each isolate in each separate broth to give a corrected OD₅₇₀. Biofilm production was classified as: no biofilm production if OD₅₇₀ was <0.135; low if 0.135-0.27; moderate if 0.27-0.54 and high if above 0.54 in agreement with previous studies (Pye et al, 2013).

### Results

The results for biofilm prevention are listed in Table 1, being MIC values for each of the ear cleaners and NAC.

**Table 1**

| **Composition** | **Dilution factors Tested** | **Minimal effective dilution factor** | **MIC** |
|---|---|---|---|
| acetic acid/boric acid | 1:2, 1:4, 1:8, 1:16, 1:32 | 1:16 | 0.125 wt.% acetic acid and 0.125 wt.% acetic acid |
| Acetic acid/lactic acid | 1:2, 1:4, 1:8 | 1:>8 (no biofilm with any dilution) | <0.0125 wt.% lactic acid and 0.25 wt.% of acetic acid |
| Tris/EDTA/chlo rhexidine | 1:2, 1:4, 1:8, 1:16, 1:32, 1:64, 1:128 | 1:64 | 0.002 wt.% chlorhexidine |
| NAC | 1:1, 1:2, 1:4, 1:10, 1:20, 1:40, 1:80 | 1:4 | 50 mg/ml |

The results for biofilm breaking are listed in Tables 2, 3 and 4. *n* is the number of isolates tested (all in triplicate), *mean* is the mean optical density value of all tests, *S.D*. is the standard deviation, *Min.* is the lowest optical density value of all tests, *Max.* is the maximum optical density value of all tests and *Median* is the median optical density value of all tests. The positive control comprises water instead of compound.

**Table 2: results for optical density tests of several dilutions of several compositions without NAC.**

| | | n | Mean | S.D. | Min. | Median | Max. |
|---|---|---|---|---|---|---|---|
| Positive control | | 14 | 0.92 | 0.48 | 0.23 | 0.85 | |
| Acetic acid/lactic acid | 1:4 | 14 | 0.62 | 0.24 | 0.29 | 0.60 | 1.10 |
| | 1:8 | 14 | 0.52 | 0.23 | 0.23 | 0.49 | 1.03 |
| | 1:16 | 14 | 0.36 | 0.14 | 0.18 | 0.35 | 0.63 |
| | 1:32 | 14 | 1.02 | 0.35 | 0.31 | 0.99 | 1.68 |
| | 1:64 | 14 | 1.05 | 0.58 | 0.22 | 0.95 | 2.30 |
| Acetic acid/boric acid | 1:4 | 14 | 0.54 | 0.19 | 0.33 | 0.50 | 0.85 |
| | 1:8 | 14 | 0.68 | 0.41 | 0.27 | 0.56 | 1.73 |
| | 1:16 | 14 | 0.36 | 0.16 | 0.10 | 0.37 | 0.61 |
| | 1:32 | 14 | 0.39 | 0.22 | 0.16 | 0.34 | 0.93 |
| | 1:64 | 14 | 0.93 | 0.44 | 0.44 | 0.80 | 1.86 |
| Tris-EDTA-chlorhexidine | 1:4 | 14 | 1.02 | 0.31 | 0.45 | 1.02 | 1.50 |
| | 1:8 | 14 | 0.86 | 0.29 | 0.40 | 0.87 | 1.32 |
| | 1:16 | 14 | -0.00 | 0.22 | -0.27 | -0.06 | 0.37 |
| | 1:32 | 14 | 0.14 | 0.25 | -0.16 | 0.10 | 0.78 |
| | 1:64 | 14 | 0.09 | 0.26 | -0.26 | 0.02 | 0.58 |
| | 1:128 | 14 | 0.60 | 0.29 | 0.22 | 0.52 | 1.16 |
| | 1:256 | 14 | 0.94 | 0.36 | 0.41 | 0.91 | 1.62 |

The data shows that the formulations according to the inventions have an effect on the biofilm breaking.

**Table 3: results for optical density tests of several dilutions of several compositions with NAC.**

| | n | Mean | S.D. | Min. | Median | Max. |
|---|---|---|---|---|---|---|
| Positive control | 14 | 0.92 | 0.48 | 0.23 | 0.85 | 2.12 |
| Acetic acid/lactic acid (1:4) + NAC (5mg/mL) | 14 | 0.53 | 0.26 | 0.21 | 0.55 | 1.00 |
| Acetic acid/lactic acid (1:8) + NAC (5mg/mL) | 14 | 0.47 | 0.20 | 0.28 | 0.38 | 0.83 |
| Acetic acid/lactic acid (1:16) + NAC (5mg/mL) | 14 | 0.44 | 0.24 | 0.18 | 0.39 | 0.99 |
| Acetic acid/lactic acid (1:32) + NAC (5mg/mL) | 14 | 0.98 | 0.42 | 0.21 | 0.87 | 2.01 |
| Acetic acid/lactic acid (1:64) + NAC (5mg/mL) | 14 | 1.01 | 0.56 | 0.06 | 0.85 | 2.16 |
| Acetic acid/boric acid (1:4) + NAC (5mg/mL) | 14 | 0.70 | 0.40 | 0.29 | 0.55 | 1.74 |
| Acetic acid/boric acid (1:8) + NAC (5mg/mL) | 14 | 0.58 | 0.32 | 0.21 | 0.48 | 1.10 |
| Acetic acid/boric acid (1:16) + NAC (5mg/mL) | 14 | 0.37 | 0.20 | 0.08 | 0.34 | 0.75 |
| Acetic acid/boric acid (1:32) + NAC (5mg/mL) | 14 | 0.35 | 0.30 | 0.05 | 0.26 | 1.19 |
| Acetic acid/boric acid (1:64) + NAC (5mg/mL) | 14 | 0.73 | 0.43 | -0.04 | 0.61 | 1.48 |
| Tris-EDTA-chlorhexidine (1:4) + NAC (5mg/mL) | 14 | 0.38 | 0.34 | -0.48 | 0.45 | 0.79 |
| Tris-EDTA-chlorhexidine (1:8) + NAC (5mg/mL) | 14 | 0.27 | 0.35 | -0.43 | 0.31 | 0.77 |
| Tris-EDTA-chlorhexidine (1:16) + NAC (5mg/mL) | 14 | 0.33 | 0.17 | -0.04 | 0.35 | 0.59 |
| Tris-EDTA-chlorhexidine (1:32) + NAC (5mg/mL) | 14 | 0.28 | 0.23 | -0.04 | 0.30 | 0.76 |
| Tris-EDTA-chlorhexidine (1:64) + NAC (5mg/mL) | 14 | 0.46 | 0.26 | 0.06 | 0.40 | 1.02 |
| Tris-EDTA-chlorhexidine (1:128) + NAC (5mg/mL) | 14 | 0.22 | 0.28 | -0.19 | 0.13 | 0.71 |
| Tris-EDTA-chlorhexidine (1:256) + NAC (5mg/mL) | 14 | 0.62 | 0.30 | 0.11 | 0.64 | 1.21 |
| Comparative: only NAC (5mg/mL) | 14 | 0.38 | 0.41 | -0.03 | 0.26 | 1.40 |

The data shows that the formulations according to the inventions have an effect on the biofilm breaking.

**Table 4: results for optical density tests of several dilutions of several compositions with varying NAC concentrations. For these tests n is the number of experiments, being 14 experiments in triplicate for each of a certain dilution factor of the compound.**

| Compound | NAC conc. | n | Mean | S.D. | Min. | Median | Max. |
|---|---|---|---|---|---|---|---|
| Acetic acid/lactic acid (1:4 + 1:8 + 1:16 + 1:32 + 1:64) | 5 | 70 | 0.69 | 0.43 | 0.06 | 0.62 | 2.16 |
| | 10 | 70 | 0.73 | 0.47 | 0.10 | 0.61 | 2.22 |
| | 20 | 70 | 0.72 | 0.43 | 0.05 | 0.68 | 2.24 |
| Acetic acid/boric acid (1:4 + 1:8 + 1:16 + 1:32 + 1:64) | 5 | 70 | 0.55 | 0.37 | -0.04 | 0.47 | 1.74 |
| | 10 | 70 | 0.57 | 0.36 | -0.01 | 0.48 | 1.87 |
| | 20 | 70 | 0.56 | 0.37 | 0.03 | 0.47 | 1.47 |
| Tris-EDTA-chlorhexidine (1:4 + 1:8 + 1:16 + 1:32 + 1:64 + 1:128 + 1:256) | 5 | 98 | 0.37 | 0.30 | -0.48 | 0.38 | 1.21 |
| | 10 | 98 | 0.50 | 0.32 | -0.18 | 0.47 | 1.36 |
| | 20 | 98 | 0.56 | 0.36 | -0.42 | 0.49 | 1.68 |

The data shows that the formulations according to the inventions have an effect on the biofilm prevention.

### CLAUSES

The following clauses define several aspects and embodiments of the teaching.
1. A composition for use in the prevention and/or dissolving of biofilm present on the skin of a domestic animal, said composition comprising EDTA or a salt thereof and chlorhexidine or a derivative thereof, preferably said composition also comprises N-acetylcysteine.
2. A composition for use in the prevention and/or dissolving of biofilm present on the skin of a domestic animal, said composition comprising acetic acid and a second acid, being boric acid and/or lactic acid, preferably said composition also comprises N-acetylcysteine.
3. The composition for use according to clause 1, the composition further comprising tris(hydroxymethyl)aminomethane (Tris).
4. The composition for use according to any one of the preceding clauses, wherein the domestic animal is a dog.
5. The composition for use according to any one of the preceding clauses, wherein the composition is for use in the ear canal.
6. The composition for use according to any one of the preceding clauses 1 and 3-5 referring back to clause 1, wherein as EDTA the disodium salt of EDTA is present.
7. The composition for use according to any one of the preceding clauses, wherein said composition is an liquid aqueous composition, preferably an ototopical solution, more preferably an ear drop.
8. The composition for use according to any one of the preceding clauses 1 and 3-7 referring back to clause 1, said composition having a pH in the range of 7.8 to 8.2.
9. The composition for use according to any one of the preceding clauses 2-7 referring back to clause 2, said composition having a pH in the range of 4.4 to 4.8.
10. A method of preventing or reducing biofilm formation on the skin of a domestic animal using the composition according to any one of the clauses 1-9, preferably wherein the biofilm is produced by *Pseudomonas aeruginosa.*

## Claims

1. A formulation for use in the prevention and/or dissolving of biofilm present on the skin of a domestic animal, said formulation comprising EDTA or a salt thereof and chlorhexidine or a derivative thereof, preferably said formulation also comprises N-acetylcysteine.

2. A formulation for use in the prevention and/or dissolving of biofilm present on the skin of a domestic animal, said formulation comprising acetic acid and a second acid, being boric acid and/or lactic acid, preferably said formulation also comprises N-acetylcysteine.

3. The formulation for use according to claim 1, the formulation further comprising tris(hydroxymethyl)aminomethane (Tris).

4. The formulation for use according to any one of the preceding claims, wherein the domestic animal is a dog.

5. The formulation for use according to any one of the preceding claims, wherein the formulation is for use in the ear canal.

6. The formulation for use according to any one of the preceding claims 1 and 3-5 referring back to claim 1, wherein as EDTA the disodium salt of EDTA is present.

7. The formulation for use according to any one of the preceding claims, wherein said formulation is an liquid aqueous formulation, preferably an ototopical solution, more preferably an ear drop.

8. The formulation for use according to any one of the preceding claims 1 and 3-7 referring back to claim 1, said formulation having a pH in the range of 6.0 to 9.0.

9. The formulation for use according to any one of the preceding claims 2-7 referring back to claim 2, said formulation having a pH in the range of 2.0 to 5.0.
